Europäisches Patentamt

**European Patent Office**

**Office européen des brevets**

(19)

(11) Publication number: **0 009 262**
A1

(12)

# EUROPEAN PATENT APPLICATION

(21) Application number: **79103627.0**

(22) Date of filing: **24.09.79**

(51) Int. Cl.³: **C 07 D 303/04, C 07 D 301/12**

(30) Priority: **25.09.78 US 945548**

(43) Date of publication of application: **02.04.80**
**Bulletin 80/7**

(84) Designated Contracting States: **BE DE FR GB IT NL**

(71) Applicant: **UNION CARBIDE CORPORATION, 270, Park Avenue, New York,N.Y. 10017 (US)**

(72) Inventor: **Clinton, Nye Atwood, Brewster Hill Road, Brewster New York (US)**

(74) Representative: **Schmied-Kowarzik, Volker, Dr. et al, Patentanwälte Dipl.-Ing. P. Wirth Dr. V. Schmied-Kowarzik Dipl.-Ing. G. Dannenberg, Dr. P. Weinhold Dr D. Gudel, Dipl.-Ing. S. Schubert, Siegfriedstrasse 8 D-8000 München 40 (DE)**

(54) Epoxidation of olefins using in situ generated hydrogen peroxide.

(57) Olefin oxides are produced by preparing hydrogen peroxide in situ by oxidizing hydroquinones followed by epoxidation of olefins over an arsenic catalyst.

EP 0 009 262 A1

This invention pertains to the oxidation of olefins to olefin oxide and more particularly to the use of hydrogen peroxide produced in situ from the oxidation of hydroquinone.

Olefin oxides or epoxides are an important class of commodity chemicals and there has been a sustained interest over the years in finding improved ways to epoxidize olefins. It is well known in the art that the most sought after epoxides, ethylene oxide and propylene oxide, are difficult to form directly from the corresponding olefins. Of the huge number of routes which have appeared in the literature only a few have been commercialized.

The most common commercial route to ethylene oxide involves direct vapor-phase oxidation of ethylene over a silver catalyst. However, similar oxidations of propylene give very low yields of epoxide.

Propylene oxide can be produced by the chlorohydrin route, i.e., propylene is reacted with hypochlorous acid to form the chlorohydrin which is then dehydrochlorinated to form the epoxide. However, to operate this route successfully a company should be basic in chlorine. Furthermore, it is difficult to separate propylene oxide from the by-products of this reaction and disposal of huge amounts of caustic and chloride waste presents a major environmental problem.

In recent years the most common approach has been to transfer oxygen to the olefin from an organic peroxide produced separately by oxidation of an appropriate substrate or formed in situ by co-oxidation of the olefin with the substrate. Examples of these processes include epoxidations of olefins by organic peracids, by organic hydroperoxides in the presence of a transition metal catalyst and by co-oxidation with aldehydes. The major defect of all these routes, from a commercial point of view, is that each involves production of relativity large amounts of a co-product. For example, in epoxidation with a peracid large amounts of the corresponding carboxylic acid are produced or with a hydroperoxide the corresponding alcohol is produced. Quite apart from the problems of possible secondary reaction of the co-product with the epoxide and/or of separation of the reaction products, the economics of the process are always dependent on finding a suitable market for the co-product since recycle is not usually economically feasible.

It is therefore an object of this invention to provide a useful and economic route to the formation of olefin oxide.

Other objects will become apparent to those skilled in the art upon a further reading of this specification.

It has now been discovered that a solution of hydrogen peroxide generated by hydroquinone oxidation can be used directly in arsenic catalyzed epoxidation of olefins without the necessity of removing the quinones and other organic products produced by the oxidation of the hydroquinone.

The oxidation of the hydroquinone is conveniently carried out in a solvent selected from the class consisting of aliphatic or cycloaliphatic ethers, aliphatic esters, chlorinated alkanes, chlorinated arenes or sulfolane. The aliphatic compounds can contain 4 to about 10 carbon atoms, the cycloaliphatic ethers can contain about 5 to 7 carbon atoms, the alkanes can contain about 1 to 8 carbon and the arenes about 6 to 10 carbon atoms.

The oxidation of the hydroquinone can be effected by sparging the solution of the hydroquinone with oxygen or an oxygen containing gas such as air or oxygen mixed with inert gases such as neon or argon or nitrogen.

The term hydroquinone includes not only hydroquinone itself, i.e., 1,4-dihydroxybenzene but also alkyl or aryl substituted hydroquinones, such as durohydroquinone, naphthahydroquinone, alkylated derivatives thereof, anthrahydroquinone and alkylated derivatives thereof such as for example those containing alkyl groups having 1 to about 4 carbon atoms, such as, 2-butylanthrahydroquinone and the like.

The oxidation of the hydroquinones to produce hydrogen peroxides is conveniently carried out in a range of about 20 to about 90°C. although this temperature range is not narrowly critical.

Representative classes of solvents include ethers, esters, alcohols, glycols and chlorinated solvents such as, chlorinated hydrocarbons and chlorinated aromatics. Specific solvents include ethanol, 2-propanol, t-butanol; methyl Cellosolve, 1,2-dimethoxy ethane, diethyl Carbitol, 1,4-dioxane, chloroform, methyl acetate, methyl Cellosolve acetate, glycol diacetate, diethylene glycol diacetate and the like. Other useful solvents include ethyl acetate, tetraethylene glycol, dimethylformamide, nitromethane, tetrahydrofuran, acetone, phenyl acetate, diphenyl Carbitol, propylene glycol diacetate, cyclohexyl acetate, triethyl phosphate, and 2,2-dimethoxy propane. Co-solvents, such as, toluene, benzene, chlorobenzene, ortho-dichlorobenzene, nitrobenzene, 1,1,2,2-tetrachloroethane, n-pentane, cyclohexane and anisole can also be used.

The epoxidation of olefins in the presence of an arsenic catalyst has been described in U.S. 3,993,673 issued to C.H. McMullen which is incorporated herein by reference.

The invention is further described in the Examples which follow. All parts and percentages are by weight unless otherwise specified.

The order of addition of the reagents is not critical in either the oxidation or epoxidation steps. Thus, for example, the olefin or the arsenic catalyst can both be present during the oxidation of the hydroquinone or either or both may be added during the oxidation or after the oxidation of the hydroquinone is complete. In any of these variations the epoxidation is carried out as described in the prior art which requires the use of purified hydrogen peroxide. To the resultant solution was then added 1 ml of 1-octene and 5 microliters of a solution containing $1.4 \times 10^{-6}$ moles of arsenic triethoxide. The sample was heated to 90°C. and after 60 minutes it was found that 0.0768 millimoles of 1,2-epoxyoctane had been produced. This represents a 77% efficiency to epoxide based on the amount of hydroquinone charged.

### EXAMPLE 2

To a 1-necked round bottom reaction flask containing 0.099 millimoles of durohydroquinone was added 1 ml of a solution prepared by mixing 20 ml of dioxane and 1 ml of undecane. To this mixture was added 5 microliters of a solution containing $1.4 \times 10^{-6}$ moles of arsenic triethoxide. The flask was filled with air and allowed to react at room temperature to form hydrogen peroxide. To this solution was then added 1 ml of 1-octene and the sample heated to 90°C. After 240 minutes

11,926

0.079 millimoles of 1,2-epoxyoctane had been produced. This represents a 79% efficiency to epoxide based on the durohydroquinone charged.

### EXAMPLE 3

To a 1-necked round bottom reaction flask containing 0.1 millimoles of durohydroquinone was added 1 ml of a solution prepared by mixing 20 ml of dioxane and 1 ml of undecane. To this mixture was added 1 ml of 1-octene and 5 microliters of a solution containing $1.4 \times 10^{-6}$ moles of arsenic triethoxide. The flask was filled with air and allowed to sit at room temperature to form hydrogen peroxide. The flask was then heated to 90°C. and after 375 minutes 0.076 millimoles of 1,2-epoxyoctane had been produced. This represents an efficiency to epoxide of 76% based on the amount of durohydroquinone charged.

### EXAMPLE 4

To a 1-necked round bottom reaction flask containing 0.1 millimoles of durohydroquinone was added 1 ml of a solution prepared by mixing 20 ml of dioxane and 1 ml of undecane along with 5 microliters of a solution containing $1.4 \times 10^{-6}$ moles of arsenic triethoxide. The flask was flushed with oxygen and sealed and allowed to react at room temperature to produce hydrogen peroxide. To this solution was added 1 ml of 1-octene and the flask was then heated to 90°C. After 255 minutes, 0.0934 millimoles of 1,2-epoxyoctane had been produced. This represents a 93.4% efficiency to epoxide based on the durohydroquinone charged.

-7-

## EXAMPLE 5

To a glass tube was added 0.1 millimoles of durohydroquinone and 1 ml of a solution prepared by mixing 20 ml of dioxane and 1 ml of undecane. To this mixture was added 6 microliters of a solution containing $1.4 \times 10^{-6}$ moles of arsenic triethoxide. The tube was flushed with oxygen, sealed and placed in a 60°C. constant temperature bath until hydrogen peroxide had formed. The tube was then opened and the contents transferred into a new tube containing 1 ml of 1-octene. The sample tube was then sealed heated to 90°C. for six hours. The tube was opened and found to contain 0.884 millimoles of 1,2-epoxyoctane. This represents an 88% efficiency to epoxide based on the amount of durohydroquinone used.

## EXAMPLE 6

A solution of 1-t-butylanthrahydroquinone was prepared by reducing 265.2 mg of 2-t-butylanthraquinone in 10 ml of methyl Cellosolve acetate with 36.5 mg of 5% palladium on carbon under an atmosphere of hydrogen. After partial reduction of the quinone, a sample was withdrawn using a syringe and the catalyst removed by filtration. Analysis of 1 ml of the solution indicated that it contained 0.0785 millimoles of 2-t-butylanthrahydroquinone. One ml of this solution was added to 1 ml of 1-octene and 5 microliters of a solution containing $1.4 \times 10^{-6}$ moles of arsenic triethoxide. This reaction mixture was then exposed to oxygen and after the oxidation step was placed in a 90°C. constant temperature bath.

11,926

After 132 minutes, 0.0595 millimoles of 1,2-epoxy-octane then formed. This represents a 76% efficiency to epoxide based on the amount of hydroquinone used.

EXAMPLE 7

A solution of 2-t-butylanthrahydroquinone was prepared by reducing 265 mg of 2-t-butylanthrahydroquinone in 10 ml of methyl Cellosolve acetate with 10.2 mg. of platinum of calcium carbonate under an atmosphere of hydrogen. After partial reduction, a sample was withdrawn and filtered under an inert atmosphere. Analysis of 1 ml of this solution indicated 0.0125 millimoles of 2-t-butylanthrahydroquinone was present. One ml of this solution was added to 1 ml of 1-octene and 5 microliters of a solution containing $1.4 \times 10^{-6}$ moles of arsenic triethoxide in a closed reaction flask. The flask was flushed with oxygen and placed on a constant temperature bath at 90°C. After 225 minutes 0.00959 millimoles of 1,2-epoxyoctane was produced. This corresponds to an efficiency of 76% based on the amount of anthrahydroquinone present.

EXAMPLE 8

A solution of durohydroquinone was prepared by reducing 327.5 mg of duroquinone in 20 ml of 1,4-dioxane with 20 mg of 5% palladium deposited on calcium carbonate under an atmosphere of hydrogen. After partial reduction a sample was withdrawn and filtered under an inert atmosphere. Analysis of 1 ml of the solution indicated that it contained 0.0785 millimoles of durohydroquinone. One ml of this solution was added to a reaction flask, flushed with air and allowed to oxidize at room temperature. To this oxidized solution was added 1 ml of 1-octene and 5 microliters of a solution containing $1.4 \times 10^{-6}$ moles of arsenic triethoxide. The flask was placed in a constant temperature bath at 90°C. and after 360 minutes 0.072 millimoles of 1,2-epoxy-octane had been produced. This corresponds to an efficiency of conversion to epoxide of 92% based on the amount of durohydroquinone present.

## EXAMPLE 9

One ml of the solution of durohydroquinone prepared in Example 8 was placed in a reaction flask and the flask flushed with oxygen and sealed. After the oxidation was complete 1 ml of 1-octene and 5 microliters of a solution containing $1.4 \times 10^{-6}$ moles of arsenic triethoxide was added thereto. The flask was heated to 90°C. After 3.5 hours it was found that 0.0983 millimoles of 1,2-epoxyoctane had formed. Analysis of the solution indicated that 0.1034 millimoles of durohydroquinone had been present indicating an efficiency of conversion to the corresponding epoxide of 95% based on the amount of durohydroquinone charged.

Although the invention had been described in its preferred forms with a certain degree of particularity, it is understood that the present disclosure had been made only by way of Example and that numerous changes can be made without departing from the spirit and scope of the invention.

—10—

WHAT IS CLAIMED IS:

1. In the arsenic catalyzed, liquid phase epoxidation of olefins with hydrogen peroxide to olefin oxides, the improvement which comprises using as the hydrogen peroxide source the product obtained by oxidizing a solution of a hydroquinone in a solvent selected from the group consisting of aliphatic or cycloaliphatic ethers, aliphatic esters, aliphatic ether-esters, chlorinated alkanes, chlorinated arenes or sulfolane wherein said aliphatic compounds contain about 4 to about 10 carbon atoms, the cycloaliphatic ethers contain 5 to 7 carbon atoms, the chlorinated alkanes contain about 1 to about 8 carbons and about 1 to 3 chlorine atoms and the chlorinated arenes contain about 6 to about 10 carbon atoms and about 1 to 3 chlorine atoms, as well as aliphatic ketones containing up to 9 C-atoms and anisole at a temperature of about 20 to about 90 °C.

2. The epoxidation of claim 1 wherein the hydroquinone is 1,4-dihydroxybenzene.

3. The epoxidation claimed in claim 1 wherein the hydroquinone is durohydroquinone.

4. The epoxidation claimed in claim 1 wherein the hydroquinone is 2-t-butylanthrahydroquinone.

5. The epoxidation claimed in claim 1 wherein the olefin is 1-octene.

11,926

6. The epoxidation claimed in claim 1 wherein the solvent is an aliphatic ester, preferably butyl cellosolve acetate.

7. The epoxidation claimed in claim 1 wherein the solvent is a cycloaliphatic ether, preferably dioxane.

8. The epoxidation claimed in claim 1 wherein the oxygen-containing gas is pure oxygen.

9. The epoxidation claimed in claim 1 wherein the oxygen-containing gas is air.

European Patent Office

**EUROPEAN SEARCH REPORT**

Application number

EP 79 10 3627

| | DOCUMENTS CONSIDERED TO BE RELEVANT | | CLASSIFICATION OF THE APPLICATION (Int. Cl. ³) |
|---|---|---|---|
| Category | Citation of document with indication, where appropriate, of relevant passages | Relevant to claim | |
| D | US - A - 3 993 673 (C.H. McMULLEN) <br> * Columns 11,12; claims * | 1,6,7 | C 07 D 303/04 <br> 30 /12 |
| | US - A - 3 806 467 (Y. WATANABE) <br> * Column 4, lines 59-75; column 5, lines 1-21 * | 1-9 | |
| | US - A - 3 642 834 (Y. SUZUKI) <br> * Whole patent * | 1,5-9 | **TECHNICAL FIELDS SEARCHED (Int.Cl. ³)** |
| | CHEMISTRY AND INDUSTRY, no. 3, 17th January 1959, pages 76-79 London, G.B. "Hydrogen Peroxide by Organic Process" <br> * Whole paper * | 1-9 | C 07 D 303/04 <br> 301/12 <br> 301/06 |
| | GB - A - 1 209 321 (I.C.I ) <br> * Whole patent * | 1-9 | |

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 14-12-1979 | ALLARD |

EPO Form 1503.1  06.78